# EUROPEAN PATENT APPLICATION

(11) **EP 2 441 441 A1**
(43) Date of publication of application: **18.04.2012**
(21) Application number: 10013660.5
(22) Date of filing: 14.10.2010
(51) Int. Cl.: A61K 9/14, A61K 31/00

(54) **A sieving method for cetyl myristate and/or cetyl palmitate**

(71) Applicant: Deva Holding Anonim Sirketi, Kucukcekmece/Istanbul (TR)
(72) Inventor: Haas, Philipp Daniel, Kucukcekmece/Istanbul (TR); Firat, Omer Faruk, Kucukcekmece/Istanbul (TR); Kandemir, Levent, Kucukcekmece/Istanbul (TR); Ozbek, Mahmut, Kucukcekmece/Istanbul (TR); Koc, Fikret, Kucukcekmece/Istanbul (TR)

(57) **Abstract**

This invention is related to a sieving method of cetyl myristate or cetyl palmitate or combination of cetyl myristate and cetyl palmitate by using screen wherein screen size is 1 mm to 1.4 mm. This invention is also directed to using of lubricants/glidants in formulations of cetyl myristate or cetyl palmitate or combination of cetyl myristate and cetyl palmitate wherein API's are sieved by screen of 1 mm to 1.4 mm.

## Description

### Technical Field

This invention is related to a sieving method of cetyl myristate or cetyl palmitate or combination of cetyl myristate and cetyl palmitate by using screen wherein screen size is 1 mm to 1.4 mm. This invention is also directed to using of lubricants/glidants in formulations of cetyl myristate or cetyl palmitate or combination of cetyl myristate and cetyl palmitate wherein API's (API:Active Pharmaceutical Ingredient) are sieved by screen of 1 mm to 1.4 mm.

### Background Art

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to US US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate.That mixture comprises by weight 95% cetyl myristate and 5% cetyl palmitate by weight In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process prepares a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstructive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention discloses that particles of cetyl myristate or cetyl palmitate or combination of cetyl palmitate and cetyl myristate are sieved by using screen wherein screen size is 1 mm to 1.4 mm. This invention also defines using of lubricants/glidants in formulations of cetyl myristate or cetyl palmitate or combination of cetyl myristate and cetyl palmitate wherein API' s are sieved by screen of 1 mm to 1.4 mm.

### Technical Problem

Cetyl myristate or cetyl palmitate or combination of cetyl myristate and cetyl palmitate can be used as active pharmaceutical ingredients (API) and/or used as dietary supplement in formulations in addition to excipient properties. However both cetyl myristate and cetyl palmitate are waxy ingredients and thus in formulation stage, they are adhered and bedaubed onto the surfaces of formulation equipments. It brings about so difficulties for formulation. Therefore it is needed that non-adhering and eligible formulations.

### Solution to Problem

It is invented that sticking problem is solved by sieving of cetyl myristate and cetyl palmitate by screen with size of 1 mm to 1.4 mm. Sieving provides that more lubricant/glidant particles are placed into the particles of API's.

It is also invented that using of lubricants in formulations of cetyl myristate or cetyl palmitate or combination of cetyl myristate and cetyl palmitate wherein API's are sieved by screen of 1 mm to 1.4 mm.

### Description of embodiments

In manufacturing pharmaceutical formulations, sieving is an ubiquitous process step. Sieving of pharmaceutical materials impact on many pharmaceutical properties. The sieving process also plays a vital role in properly lubricant distribution into particles of active pharmaceutical ingredient.

The lubrication properties are correlated with surface area. Sieving increases the surface area and thus lubricant particles more interpose between particles of active pharmaceutical ingredient. If sieving is not executed and API is waxy, lubricant particles can't be placed into particles of API and its waxy properties can't be removed.

In one aspect of this invention is to provide a sieving method of cetyl myristate or cetyl palmitate or combination of cetyl myristate and cetyl palmitate wherein API's are screened by a sieve with 1 mm to 1.4 mm to obtain eligible and elegant formulations.

In another aspect of this invention to obtain eligible and elegant formulations, a lubricant or mixtures of lubricants are used in formulations of cetyl myristate or cetyl palmitate or combination of cetyl myristate and cetyl palmitate wherein API's are sieved by screen of 1 mm to 1.4 mm. Herein the term of lubricant or glidant are used in same meaning.

If cetyl myristate or cetyl palmitate or combination of cetyl myristate and cetyl palmitate are sieved a screen lower than with 1 mm, flowability of active pharmaceutical ingredient(s) are dissappeared. If cetyl myristate or cetyl palmitate or combination of cetyl myristate and cetyl palmitate are sieved a screen greater than with 1,4 mm, bedaubing problem is appeared.

Accordingly, in formulations of cetyl myristate or cetyl palmitate or combination of cetyl myristate and cetyl palmitate,which are sieved by screen of 1 mm to 1.4 mm and lubricant is used in the range of from about 0.1 % to about 15 % by weight of pharmaceutical composition.

Preferably lubricant is used in the range of from about 1 % to about 5 % by weight of pharmaceutical composition.

More preferably lubricant is used about 2,3 % by weight of pharmaceutical composition.

In another aspect it is also invented that, to obtain eligible and elegant formulations, lubricant should be in a weight ratio to active ingredients in a pharmaceutical or dietary supplement composition. Accordingly weight ratio of lubricant to active ingredient is from 1:7 to 1:80 .Preferably weight ratio is 1:35.

Yet another aspect of this invention is to provide an eligible and elegant formulation wherein lubricant is in a weight ratio to other non-active pharmaceutical ingredient from 1:1 to 1:15:, preferably 1:6.

In accordance with invention it also invented that lubricant should be in a weight ratio of from 1:7 to 1:70 to total pharmaceutical composition. Preferably weight ratio is 1:42.

In formulations, binders,plasticizers and fillers can be used along with lubricants/glidants.

Lubricants/Glidants are, but not limited to, magnesium stearate,calcium stearate,hydrogenated castor oil,glyceryl behenate,glyceryl monostearate,glyceryl palmitostearate,leucine,mineral oil, light mineral oil, myristic acid,palmitic acid,polyethylene glycol,potassium benzoate,sodium benzoate,sodium lauryl sulfate,sodium stearyl fumarate,stearic acid,talc, hydrogenated vegetable oil,zinc stearate, magnesium lauryl sulphate,sodium stearyl fumarate, polyethylene glycol, stearic acid, colloidal silicon dioxide or mixtures thereof and the like. Preferred lubricant is magnesium stearate. Two different kinds of lubricants are preferred to be benefited from different proporties of lubrication.

Binders are, but not limited to, sodium alginate, cellulose, methylcellulose, ethylcellulose, hydroxypropyl cellulose, hydroxypropyl methylcellulose, sodium carboxymethyl cellulose, polypropylpyrrolidone, polyvinylprrolidone, gelatin, polyethylene glycol, starch, pre-gelatinized starch, sugars, trehalose, glucose,tragacanth, sorbitol, acacia, alginates, carrageenan, xanthan gum, locust bean gum and gum arabic, waxes,polyacrylamide , mixtures thereof, and the like.

Diluents/fillers are, but not limited to, mannitol, sorbitol, xylitol, microcrystalline cellulose, silicified microcrystalline cellulose, hydroxypropyl methylcellulose, hydroxypropyl cellulose, pullulan and fast dissolving carbohydrates such as Pharmaburst™, mixtures thereof and the like.

Plasticizers are , but not limited to, polyethylene glycol, propylene glycol, triacetin, dimethyl phthalate, diethyl phthalate, dibutyl phthalate, dibutyl sebacate, triethyl citrate, tributyl citrate, triethyl acetyl citrate, castor oil , acetylated monoglycerides, mixtures thereof and the like. Plasticizer is used to have hard granulate.

### Example

**Table 1**

| INGREDIENTS | mg |
|---|---|
| Cetyl Myristate/API | 333.3 |
| Cetyl Palmitate/API | 16.7 |
| Diluent/Filler | 45 |
| Binder | 10 |
| Plasticizer | 5 |
| Lubricant/Glidant | 10 |
| TOTAL | 420.0 |

## Claims

1. A pharmaceutical or dietary supplement composition comprising (a) cetyl myristate, or (b) cetyl palmitate or (c) cetyl myristate and cetyl palmitate combination **characterized in that** said active ingredient or ingredients are sieved from a screen with 1 mm to 1.4 mm and wherein lubricant/glidant or mixtures of lubricants/glidants are a) in the range of from 0.1 % to 15 % , preferably from 1 % to 5 %,more preferably 2,3 % by weight of composition or b) in a weight ratio to active ingredients from 1:7 to 1:80, preferably 1:35 or c) in a weight ratio to other non-active pharmaceutical ingredients from 1:1 to1:15, preferably 1:6, d) in a weight ratio to total pharmaceutical composition from 1:7 to 1:70 ,preferably 1:42.
